# EUROPEAN PATENT SPECIFICATION

(11) **EP 2 345 887 B1**
(45) Date of publication and mention of the grant of the patent: **20.05.2020**
(21) Application number: 10195074.9
(22) Date of filing: 15.12.2010
(51) Int. Cl.: G01N 21/25, G01N 21/898, G01N 21/64

(54) **Process and apparatus for determining the properties of a piece of timber**
Verfahren und Vorrichtung zur Bestimmung der Eigenschaften eines Holzstücks
Procédé et appareil pour déterminez les propriétés d'une piece de bois

(30) Priority: 15.12.2009 FI 20096322
(43) Date of publication of application: 20.07.2011
(73) Proprietor: UPM-Kymmene Corporation, 00100 Helsinki (FI)
(72) Inventor: Hämäläinen, Sampsa, 15900, Lahti (FI); Hyvönen, Petri, 07700, Koskenkylän saha (FI); Antikainen, Jukka, 80130, Joensuu (FI); Kinnunen, Jussi, 80160, Joensuu (FI)
(74) Representative: Papula Oy

(56) References cited:
- WO-A1-2008/031621
- DE-A1- 4 343 058
- DE-C1- 19 609 045
- US-A- 3 976 384
- DATABASE WPI Week 200276 Thomson Scientific, London, GB; AN 2002-705035 XP002775178, -& SE 0 004 304 L (TRAETEK AB) 23 May 2002 (2002-05-23)
- S T Sum ET AL: "Laser-excited fluorescence spectra of eastern SPF wood species. An optical technique for identification and separation of wood species?", Wood Science and Technology, 1 September 1991 (1991-09-01), pages 405-413, XP055035193, DOI: 10.1007/BF00225233 Retrieved from the Internet: URL:http://www.springerlink.com/content/w1 56w98707184204/fulltext.pdf [retrieved on 2012-08-09]
- STEPHANIE T. DYER: "Wood Fluorescence of Indigenous South African Trees", IAWA JOURNAL, vol. 9, no. 1, 1 January 1988 (1988-01-01) , pages 75-87, XP055419917, NL ISSN: 0928-1541, DOI: 10.1163/22941932-90000472

## Description

### FIELD OF THE INVENTION

The invention relates to a process and an apparatus for determining the properties of a piece of timber in connection with the manufacture of wood products.

### BACKGROUND OF THE INVENTION

For wood quality control, different properties are determined from the wood, e.g. the wood species, the proportion of heartwood, the proportion of sound wood and the occurrence of blue stain fungus. Conventionally, the pieces of wood have been sorted visually by people in different stages, e.g. in the sorting of logs or in the sawing process. Lately, the sorting has been started to perform mechanically and automatically. A problem has been to find suitable sorting processes in order reliably to determine the desired properties from a piece of wood. It is known that the wood product technology and different determination processes, e.g. sorting by camera, have until now been based on solutions that operate mainly in the visible region. In the visible region it is not possible to determine all desired properties of pieces of wood with sufficient accuracy.

Known from reference WO 2008/031621 is a method where the surface of a piece of wood is illuminated and the illuminated area is observed by a camera. In the method, concrete defects, such as knot points and bores, are determined from the piece of wood.

From DE 19609045, DE 4343058, SE 0004304 and US 3976384 are known different methods for determining properties from wood samples. Further, S.T. Sum et al., "Laser-excited fluorescence spectra of eastern SPF wood species. An optical technique for identification and separation of wood species?", Wood Science and Technology, 1 January 1991, pages 405-413, and Stephanie T. Dyer, "Wood Fluorescence of Indigenous South African Trees", IAWA JOURNAL, vol.9, no.1, 1 January 1988, pages 75-87, disclose a use of fluorescence for determining properties from wood samples.

### OBJECTIVE OF THE INVENTION

An objective of the invention is to resolve the preceding problems and disclose a new type of, easy to use and reliable process and apparatus for determining the properties of a piece of timber in connection with logs, timber products and reprocessed wood products.

### SUMMARY OF THE INVENTION

The process and apparatus according to the invention are characterized by what has been presented in the claims.

The invention is based on a process for determining the properties of a piece of timber in connection with the manufacture of wood products wherein the piece of timber is a part sawn from a log, or a wood product formed by sawing, or a further treated wood product, wherein a wood sample is selected from the piece of timber, the wood sample is illuminated by a light source, resulting signals are observed and analyzed from the wood sample illuminated by the light source, and a property of the wood sample is determined by the signals. According to the invention the wood sample is the cross-sectional surface of one end of the piece of timber; the wood sample is illuminated by a UV light source and visible light source; the light is focused on the cross-sectional surface of the piece of timber; the resulting signals are observed and analyzed from the wood sample illuminated by the UV light based on fluorescence differences and from the wood sample illuminated by the visible light based on color differences; and the properties selected from the group of decay, rot fungus, blueness, site of heartwood, amount of heartwood, location of pith, amount of sapwood, location of the piece of timber measured from one end of the piece, e.g. a heart, middle or sap piece, and combinations of the preceding properties are determined from the wood sample by the signals such that the decay, rot fungus and blueness are determined by means of signals based on the fluorescence differences and the color differences, and the site of heartwood, amount of heartwood, location of pith, amount of sapwood and location of the piece of timber on a log are determined by means of signals based on the fluorescence differences.

In addition, the invention is based on an apparatus for determining the properties of a piece of timber in connection with the manufacture of wood products wherein the apparatus includes at least one lighting unit in order to illuminate a wood sample which is the piece of timber and in which the piece of timber is a part sawn from a log, or a wood product formed by sawing, or a further treated wood product, at least one observation means in order to observe and measure the resulting signals from the illuminated wood sample, analysis means in order to analyze the resulting signals, and determination means in order to determine the properties of the piece of timber on the basis of the signals. According to the invention the apparatus includes at least one lighting unit comprising a light source based on UV light and at least one lighting unit comprising a light source based on visible light in order to illuminate the wood sample and in order to focus the light on the wood sample which is a cross-sectional surface of one end of the piece of timber, analysis means is arranged to analyze the resulting signals based on fluorescence differences in the wood sample illuminated by UV light and on color differences in the wood sample illuminated by visible light, and determination means is arranged to determine the properties of the piece of timber selected from the group of decay, rot fungus, blueness, site of heartwood, amount of heartwood, location of pith, amount of sapwood, location of the piece of timber, e.g. a heart, middle or sap piece, and their combinations, on the base of the measured signals such that the decay, rot fungus and blueness are determined by means of signals based on the fluorescence differences and the color differences, and the site of heartwood, amount of heartwood, location of pith, amount of sapwood and location of the piece of timber on a log are determined by means of signals based on the fluorescence differences.

In one preferred embodiment a color signal is detected, e.g. the black/white/gray tones or all colors, as a result of illuminating the wood sample. The color signal is the product of radiation spectrum of the light source and reflection spectrum of the wood sample. All wavelengths of the color signal are not detected equally sensitively depending on the observation means.

The invention is specifically based on a combination where a wood sample from a piece of timber can be illuminated by UV light and visible light as desired and many desired properties can be simultaneously determined from the wood sample.

A piece of timber in this context stands for any log to be sawn or a part of such a log or any part sawn from a log, e.g. the heart section, i.e. a piece of the central section, a surface board part or equivalent part, or a wood product formed by sawing, such as a board, plank, beam, cant, batten, lath, veneer or equivalent, or a further treated wood product, such as a reprocessed, seasoned, planed, sanded and/or finished wood product.

A wood sample in this context stands for any complete piece of timber or a part thereof which is illuminated, analyzed and determined for the desired properties.

Heartwood in this context stands for dead tissue in the center part of a stem.

A face in this context stands for a surface face, heart face or edges of a piece of timber.

According to the invention the wood sample is the cross-sectional surface of a piece of timber, i.e. one end, on which the light is focused and from which the desired properties are determined.

In one embodiment of the invention the wood sample is the face or the edge of a piece of timber on which the light is focused and from which the desired properties are determined. In one embodiment the face is measured.

Preferably an illuminated wood sample is observed or imaged by an observation means, such as a camera, from the same site on which the light is focused.

In one embodiment of the invention a wood sample is illuminated sequentially by a UV light source and a visible light source. As UV light and visible light sources, any UV light and visible light source known per se and suitable for the purpose of use can be used, e.g. different lamps, fluorescent lamps or led lights.

In one embodiment of the invention a lighting unit includes 1 to 5 parallel light sources. There may be many lighting units or the lighting unit may include many light sources. The number of light sources depends on the size, such as length, of a wood sample to be determined.

In one embodiment of the invention the observation means includes 1 to 5 parallel observation devices. There may be many observation means or the observation means may include many observation devices. The number of observation devices depends on the size, such as length, of a wood sample to be determined. In one embodiment the observation means or the observation device observes, e.g. takes and image of, a wood sample at a substantially right angle relative to the wood sample. In another embodiment the observation means or the observation device observes a wood sample at an angle other than right angle relative to the wood sample.

In one embodiment the observation device is a detector, e.g. in order to observe fluorescence and fluorescence differences in the UV region. In one embodiment the observation device is a camera, e.g. in order to observe fluorescence and fluorescence differences in the UV region or to observe color differences in the visible region. In one embodiment the observation device is a device based on machine vision. The camera may be any camera or camera device known per se and suitable for sorting by camera. In one embodiment the selected camera is based on electronic camera technology known per se. The camera or the detector may be based on a CCD or a CMOS cell and technology. In one embodiment the camera is a UV sensitive camera. In one embodiment the camera is a color camera. In one embodiment the camera is an RGB camera. In one embodiment the camera is a black-and-white camera or a grayscale camera. In one embodiment the camera is a high-speed camera.

In one embodiment of the invention a wood sample is illuminated by UV light, and fluorescence and fluorescence differences are observed by a detector. In one embodiment a wood sample is illuminated by UV light, and fluorescence and fluorescence differences are observed by a camera. In one embodiment a wood sample is illuminated by visible light, and color differences are observed by a camera.

In one embodiment the visible wavelength region is between 380 and 780 nm. In one embodiment of the invention the UV wavelength is selected from between 300 and 450 nm, in one preferred embodiment between 300 and 340 nm.

By selecting the suitable wavelength region and especially the combination of suitable wavelength regions, several different properties, e.g. physical, structural, biological, biomechanical, mechanical or equivalent properties, can be simultaneously determined from a piece of timber. It was unexpectedly discovered that several desired properties of a piece of timber can be better determined in the UV region than in other light regions. In addition, it was discovered that determination of the properties can be complemented with measurements in the visible region, i.e. the best combination in determining the properties of a piece of timber is achieved with a combination of measurements in the UV region and visible region.

In one embodiment of the invention the apparatus includes a filter in order to improve and/or amplify the signal. In one embodiment the filter is located in front of the observation means. In one embodiment the filter is an interference filter.

In one embodiment the fluorescence differences are amplified by a filter. In one embodiment the filter is arranged to transmit UV radiation in the region of approximately 400 to 500 nm, because it was discovered that fluorescence resolution is very good in this region.

In one embodiment of the invention the employed analysis and determination means encompasses computers and/or computer software suitable for the purpose of use and having a sufficient capacity in order to process and analyze the measurement data, such as signals, and additionally to determine the properties of a piece of timber on the basis of measurement data. In one preferred embodiment the computer software is integrated with a logistics system of the factory or sawmill.

In different embodiments of the invention it is possible to use any suitable UV light sources, visible light sources, detectors, cameras, devices based on machine vision, filters, computers and data processing software and systems that are known per se and can be adapted to be suitable for the purpose of use.

In one embodiment the blueness and decay are determined by a two-stage process by measuring a wood sample illuminated by UV light in the first stage and measuring the same wood sample illuminated by visible light in the second stage. In one embodiment the result is provided by combination of image information from the two stages. The blue stain fungi are fluorescent but decayed wood is not fluorescent. Different intensity areas are identified from signals, e.g. image, of the first stage due to fluorescence differences. The fluorescent areas appear as bright and the non-fluorescent as non-bright areas. Thus, the non-bright areas represent decayed wood and the bright areas represent sound wood and wood affected by blue stain fungus, because both produce fluorescence, yet different types of fluorescence. The color differences are identified from signals, e.g. image, of the second stage. In color difference changes an early hard rot is represented by moderate color difference changes compared with sound wood, and further advanced rot and blue stain fungus are represented by the dark areas diverging from sound wood through color difference change. Hence, the blueness can be determined from combinations of signals from the two stages. Preferably a measuring event illuminated by UV light is sufficient to measure decayed wood.

In one embodiment the proportion/amount of heartwood, the proportion/amount of sapwood and/or an equivalent property is determined from the cross-sectional surface of a piece of timber based on the fluorescence of UV light. In one embodiment the site of pith and/or the location of the wood sample is determined based on the fluorescence of UV light. The observation means, such as a camera, is used to take an image of the cross-sectional surface illuminated by UV light in order to determine the proportion or amount of heartwood and/or sapwood. The heartwood is differentiated from sapwood on the basis of fluorescence differences because the heartwood is more fluorescent than sapwood.

In an example which does not form part of the invention the dead knot, sound knot, cracks, bores, dirt, wane or an equivalent property is determined from a piece of timber on the basis of measurement based on fluorescence (illuminated by UV light) or based on color differences (illuminated by visible light) or on the basis of a combined measurement of the above-said.

In one embodiment of the invention the process is used in the sorting of pieces of timber, such as logs and wood products. In one embodiment the process is used in connection with a sawing process. In one embodiment the process is used in a log rotating process. In one embodiment the process is used in connection with a finishing process of wood products, e.g. in sorting on the basis of color, evenness of color, spreading, evenness or amount of the finishing material.

The process and apparatus can be applied in the track speeds used in the industry. In addition, the process and apparatus can be used in connection with the processing of any wood material.

The process and apparatus according to the invention provide considerable advantages as compared to the prior art.

Thanks to the invention, a process and an apparatus that lend themselves to the identification of defects in logs, unseasoned products and seasoned wood products is provided. In addition, the invention lends itself to the identification of defects that appear at the ends of a log or other piece of timber.

Thanks to the invention, an industrially applicable easy, quick, reliable and useful process for sorting wood products is provided. The process and apparatus according to the invention can also be applied at high track speeds.

### LIST OF FIGURES

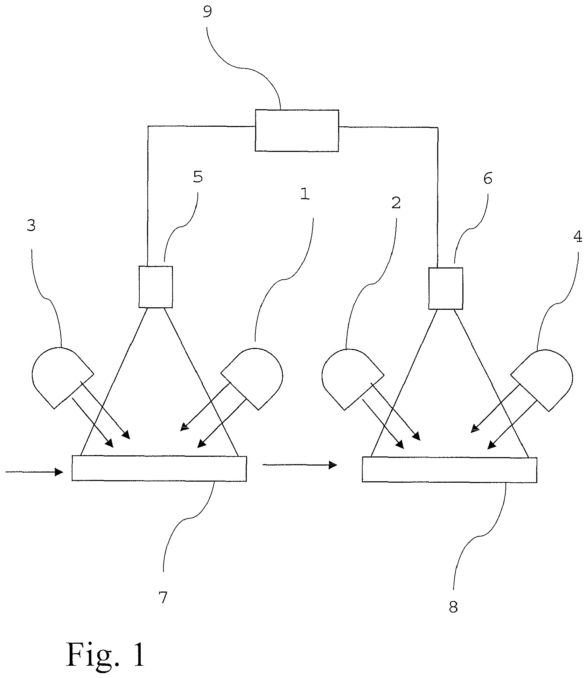

Fig. 1 presents one apparatus according to the invention as a simplified diagram.

### DETAILED DESCRIPTION OF THE INVENTION

In the following, the invention will be described by detailed exemplary embodiments with reference to the accompanying figure.

### Example 1

Fig. 1 presents the apparatus according to the invention including two fluorescent lamp lighting units 1, 2, the first 1 being based on UV light and the second 2 on visible light in order to illuminate a wood sample 7, 8 of a piece of timber. Both lighting units 1, 2 include two light sources 3,4. The apparatus includes two cameras 5, 6 in order to observe and measure the resulting signals from the illuminated wood sample 7, 8, the cameras 5, 6 being integrated in connection with the lighting units 1, 2. The employed cameras 5, 6 are cameras based on a CCD cell. In front of the lens of the camera 5 that takes an image of the sample 7 illuminated by UV light there is an interference filter (not shown in the figure) that transmits in the region of 400 to 500 nm. Furthermore, the apparatus includes analysis means 9 in order to analyze the resulting signals based on fluorescence differences in the wood sample 7 illuminated by UV light and on a color difference in the wood sample 8 illuminated by visible light, and determination means 9 in order to determine the properties of the piece of timber selected from the group of decay, rot fungus, blueness, dead or sound knot, dirt, site of heartwood, amount of heartwood, location of pith, amount of sapwood, location of the piece of timber on a log and their combinations, by the analyzed signals. The analysis and determination means 9 encompass a computer and suitable commercial software therein.

### Example 2

In this experiment the differentiation between heartwood and sapwood and the proportion of heartwood from a piece of pine wood timber were examined. Light in the UV region was focused at one end of the piece of timber, and an image was taken of the end by an end camera, which was a gray scale camera. From the image, the proportion of heartwood was identified based on fluorescence differences of the UV light, because the heartwood and sapwood are differently fluorescent. The UV light used in the measurements was light that has a peak at the wavelength of 320nm. In front of the camera there was an interference filter in order to raise the intensity difference between sap- and heartwood.

In addition, a comparison was performed in the experiment by determining the proportion of heartwood manually, by iodine and by the process according to the invention. It was discovered that the results were at the same level in determinations by all methods.

In the experiments it was discovered that the determination of the proportion of heartwood was very quick and easy by the process according to the invention. The measured heartwood quantities corresponded to the actual piece. It was discovered that heartwood had a strong fluorescence in comparison with sapwood under UV lighting.

In addition, it was discovered that the process according to the invention could be used with high track speeds.

### Example 3

In this experiment the determination of blueness and decay from a piece of timber by an apparatus equivalent to the apparatus presented in Fig. 1 was examined. The measurement was taken from the face of the piece of timber.

The blueness and decay were determined by a two-stage process by taking an image of a wood sample illuminated by UV light with a CCD camera in the first stage and taking an image of the same wood sample illuminated by visible light in the second stage. The image of the wood sample illuminated by visible light was taken with an RGB camera.

The result was provided by combination of image information from the two stages. The blue stain fungi are fluorescent but the decayed wood is not fluorescent. Different intensity areas in terms of darkness were identified from the image of the first stage due to the fluorescence differences. The fluorescent areas appeared as bright and the non-fluorescent as dark. Thus, the dark areas represented decayed wood and the bright areas represented sound wood and wood affected by blue stain fungus, because both produce fluorescence, yet different types of fluorescence. The decayed areas of the wood and those in the process of decaying could be computed from the image of the first stage. The color differences were identified from the image of the second stage. The dark areas represented decayed wood and wood affected by blue stain fungus because both were darker than normal wood under visible light. Thus, the blueness could be determined from combinations of signals from the two stages, i.e. by comparing the images from both stages. The sorting could be performed out of the differences of the intensities in the image pixels.

Simultaneously in connection with the determination of blueness and decay, also other properties could be determined from the wood sample, e.g. the proportion of heartwood from the sample illuminated by UV light. The sorting could be performed by the intensity differences of image pixels.

In the experiments it was discovered that the determination of blueness and decay was very quick, easy and reliable by the process according to the invention. Furthermore, it was discovered that it was easy to determine several other properties from the wood sample simultaneously in connection with the determination of blueness and decay by the process and apparatus in question. In addition, it was discovered that the process according to the invention could be used with high track speeds.

The process and the apparatus according to the invention lend themselves as different embodiments to be used in the sorting of the most different kinds of pieces of timber and in connection with the manufacture of wood products.

The invention is not limited merely to the examples referred to above; instead, many variations are possible within the scope of the inventive idea defined by the claims.

## Claims

1. A process for determining the properties of a piece of timber wherein the piece of timber is a part sawn from a log, or a wood product formed by sawing, or a further treated wood product, wherein a wood sample is selected from the piece of timber, the wood sample is illuminated by a light source, resulting signals are observed and analyzed from the wood sample illuminated by the light source, and a property of the wood sample is determined by the signals, wherein the wood sample is a cross-sectional surface of one end of the piece of timber; the wood sample is illuminated by a UV light source and visible light source; the light is focused on the cross-sectional surface of the piece of timber; the resulting signals are observed and analyzed from the wood sample illuminated by the UV light based on fluorescence differences and from the wood sample illuminated by the visible light based on color differences; and the properties selected from the group of decay, rot fungus, blueness, site of heartwood, amount of heartwood, location of pith, amount of sapwood, location of the piece of timber on a log and their combinations are determined from the wood sample by the signals such that the decay, rot fungus and blueness are determined by means of signals based on the fluorescence differences and the color differences, and the site of heartwood, amount of heartwood, location of pith, amount of sapwood and location of the piece of timber on a log are determined by means of signals based on the fluorescence differences.

2. The process according to claim 1, **characterized in that** the wood sample is sequentially illuminated by the UV light source and the visible light source.

3. The process according to claim 1 or 2, **characterized in that** the wood sample is illuminated by UV light, and fluorescence and fluorescence differences are observed by a detector.

4. The process according to any one of claims 1 to 3, **characterized in that** the wood sample is illuminated by UV light, and fluorescence and fluorescence differences are observed by a camera.

5. The process according to any one of claims 1 to 4, **characterized in that** the wood sample is illuminated by visible light, and color differences are observed by a camera.

6. The process according to any one of claims 1 to 5, **characterized in that** the signal is amplified by a filter.

7. The process according to any one of claims 1 to 6, **characterized in that** the wavelength of UV light is selected from the range of 300 to 450 nm.

8. The process according to any one of claims 1 to 7, **characterized in that** the process is used in the sorting of pieces of timber.

9. An apparatus for determining the properties of a piece of timber wherein the apparatus includes at least one lighting unit (1,2) in order to illuminate a wood sample (7,8) which is the piece of timber and in which the piece of timber is a part sawn from a log, or a wood product formed by sawing, or a further treated wood product, at least one observation means (5,6) in order to observe and measure the resulting signals from the illuminated wood sample (7,8), analysis means (9) in order to analyze the resulting signals, and determination means (9) in order to determine the properties of the piece of timber on the basis of the signals, wherein the apparatus includes at least one lighting unit (1) comprising a light source based on UV light (3) and at least one lighting unit (2) comprising a light source based on visible light (4) in order to illuminate the wood sample (7, 8) and in order to focus the light on the wood sample which is a cross-sectional surface of one end of the piece of timber, analysis means (9) is arranged to analyze the resulting signals based on fluorescence differences in the wood sample illuminated by UV light and on color differences in the wood sample illuminated by visible light, and determination means (9) is arranged to determine the properties of the piece of timber selected from the group of decay, rot fungus, blueness, site of heartwood, amount of heartwood, location of pith, amount of sapwood, location of the piece of timber on a log and their combinations on the basis of the signals such that the decay, rot fungus and blueness are determined by means of signals based on the fluorescence differences and the color differences, and the site of heartwood, amount of heartwood, location of pith, amount of sapwood and location of the piece of timber on a log are determined by means of signals based on the fluorescence differences.

10. The apparatus according to claim 9, **characterized in that** the lighting unit (1,2) includes 1 to 5 light sources (3,4).

11. The apparatus according to claim 9 or 10, **characterized in that** the observation means includes 1 to 5 observation devices (5,6).

12. The apparatus according to any one of claims 9 to 11, **characterized in that** the apparatus includes a detector (5,6) as an observation device.

13. The apparatus according to any one of claims 9 to 12, **characterized in that** the apparatus includes a camera (5,6) as an observation device.

14. The apparatus according to any one of claims 9 to 13, **characterized in that** the apparatus includes a filter in order to amplify the signal.

15. The apparatus according to any one of claims 9 to 14, **characterized in that** the apparatus includes computer software (9) as an analysis and determination means.

## Patentansprüche

1. Verfahren zur Bestimmung der Eigenschaften eines Stücks Nutzholz, wobei das Stück Nutzholz ein aus einem Baumstamm gesägter Teil, oder ein durch Sägen geformtes Holzprodukt, oder ein weiter behandeltes Holzprodukt, ist, wobei aus dem Stück Nutzholz eine Holzprobe ausgewählt wird, die Holzprobe von einer Lichtquelle beleuchtet wird, resultierende Signale aus der von der Lichtquelle beleuchteten Holzprobe beobachtet und analysiert werden und eine Eigenschaft der Holzprobe durch die Signale bestimmt wird, wobei die Holzprobe eine Querschnittsfläche eines Endes des Stücks Nutzholz ist; die Holzprobe von einer Lichtquelle für UV-Licht und einer Lichtquelle für sichtbares Licht beleuchtet wird; das Licht auf die Querschnittsfläche des Stücks Nutzholz fokussiert wird; die resultierenden Signale von der mit dem UV-Licht beleuchteten Holzprobe auf Grundlage von Fluoreszenzunterschieden und von der mit dem sichtbaren Licht beleuchteten Holzprobe auf Grundlage von Farbunterschieden beobachtet und analysiert werden; und die Eigenschaften ausgewählt aus der Gruppe von Verfall, Fäulnispilz, Bläue, Lage des Kernholzes, Menge des Kernholzes, Ort der Markröhre, Menge des Splintholzes, Lokalisierung des Stücks Nutzholz auf einem Baumstamm und deren Kombinationen von der Holzprobe durch die Signale bestimmt werden, sodass der Verfall, der Fäulnispilz und die Bläue anhand von Signalen bestimmt werden, die auf den Fluoreszenzunterschieden und den Farbunterschieden basieren, und die Lage des Kernholzes, die Menge des Kernholzes, der Ort der Markröhre, die Menge des Splintholzes und die Lokalisierung des Stücks Nutzholz auf einem Baumstamm anhand von Signalen bestimmt werden, die auf den Fluoreszenzunterschieden basieren.

2. Verfahren nach Anspruch 1, **dadurch gekennzeichnet, dass** die Holzprobe nacheinander von der Lichtquelle für UV-Licht und der Lichtquelle für sichtbares Licht beleuchtet wird.

3. Verfahren nach Anspruch 1 oder 2, **dadurch gekennzeichnet, dass** die Holzprobe durch UV-Licht beleuchtet wird und Fluoreszenz und Fluoreszenzunterschiede durch einen Detektor beobachtet werden.

4. Verfahren nach einem der Ansprüche 1 bis 3, **dadurch gekennzeichnet, dass** die Holzprobe durch UV-Licht beleuchtet wird und Fluoreszenz und Fluoreszenzunterschiede durch eine Kamera beobachtet werden.

5. Verfahren nach einem der Ansprüche 1 bis 4, **dadurch gekennzeichnet, dass** die Holzprobe durch sichtbares Licht beleuchtet wird und Farbunterschiede durch eine Kamera beobachtet werden.

6. Verfahren nach einem der Ansprüche 1 bis 5, **dadurch gekennzeichnet, dass** das Signal durch einen Filter verstärkt wird.

7. Verfahren nach einem der Ansprüche 1 bis 6, **dadurch gekennzeichnet, dass** die Wellenlänge von UV-Licht aus dem Bereich von 300 bis 450 nm ausgewählt wird.

8. Verfahren nach einem der Ansprüche 1 bis 7, **dadurch gekennzeichnet, dass** das Verfahren bei der Sortierung von Stücken von Nutzholz verwendet wird.

9. Vorrichtung zur Bestimmung der Eigenschaften eines Stücks Nutzholz, wobei die Vorrichtung
zumindest eine Beleuchtungseinheit (1, 2), um eine Holzprobe (7, 8) zu beleuchten, die das Stück Nutzholz ist und in der das Stück Nutzholz ein aus einem Baumstamm gesägter Teil, oder ein durch Sägen geformtes Holzprodukt, oder ein weiter behandeltes Holzprodukt ist,
zumindest ein Beobachtungsmittel (5, 6), um die resultierenden Signale aus der von der Lichtquelle beleuchteten Holzprobe (7, 8) zu beobachten und zu messen, ein Analysemittel (9), um die resultierenden Signale zu analysieren und
ein Bestimmungsmittel (9), um die Eigenschaften der Holzprobe auf Grundlage der Signale zu bestimmen,
enthält,
wobei die Vorrichtung
zumindest eine Beleuchtungseinheit (1), die eine Lichtquelle auf Grundlage von UV-Licht (3) umfasst, und zumindest eine Beleuchtungseinheit (2), die eine Lichtquelle auf Grundlage von sichtbarem Licht (4) umfasst, um die Holzprobe (7, 8) zu beleuchten und um das Licht auf die Holzprobe, die eine Querschnittsfläche eines Endes des Stücks Nutzholz ist, zu fokussieren, enthält
wobei ein Analysemittel (9) angeordnet ist, um die resultierenden Signale auf Grundlage von Fluoreszenzunterschieden in der durch UV-Licht beleuchteten Holzprobe und von Farbunterschieden in der durch sichtbares Licht beleuchteten Holzprobe zu analysieren, und
wobei ein Bestimmungsmittel (9) angeordnet ist, um die Eigenschaften des Stücks Nutzholz, die aus der Gruppe von Verfall, Fäulnispilz, Bläue, Lage des Kernholzes, Menge des Kernholzes, Ort der Markröhre, Menge des Splintholzes, Lokalisierung des Stücks Nutzholz auf einem Baumstamm und deren Kombinationen ausgewählt sind, basierend auf den Signalen zu bestimmen, sodass der Verfall, der Fäulnispilz und die Bläue anhand von Signalen bestimmt werden, die auf den Fluoreszenzunterschieden und den Farbunterschieden basieren, und die Lage des Kernholzes, die Menge des Kernholzes, der Ort der Markröhre, die Menge des Splintholzes und die Lokalisierung des Stücks Nutzholz auf einem Baumstamm anhand von Signalen bestimmt werden, die auf den Fluoreszenzunterschieden basieren.

10. Vorrichtung nach Anspruch 9, **dadurch gekennzeichnet, dass** die Beleuchtungseinheit (1, 2) 1 bis 5 Lichtquellen (3, 4) beinhaltet.

11. Vorrichtung nach Anspruch 9 oder 10, **dadurch gekennzeichnet, dass** das Beobachtungsmittel 1 bis 5 Beobachtungseinrichtungen (5, 6) beinhaltet.

12. Vorrichtung nach einem der Ansprüche 9 bis 11, **dadurch gekennzeichnet, dass** die Vorrichtung einen Detektor (5, 6) als eine Beobachtungseinrichtung beinhaltet.

13. Vorrichtung nach einem der Ansprüche 9 bis 12, **dadurch gekennzeichnet, dass** die Vorrichtung eine Kamera (5, 6) als eine Beobachtungseinrichtung beinhaltet.

14. Vorrichtung nach einem der Ansprüche 9 bis 13, **dadurch gekennzeichnet, dass** die Vorrichtung einen Filter beinhaltet, um das Signal zu verstärken.

15. Vorrichtung nach einem der Ansprüche 9 bis 14, **dadurch gekennzeichnet, dass** die Vorrichtung Computersoftware (9) als ein Analyse- und Bestimmungsmittel beinhaltet.

## Revendications

1. Procédé pour déterminer les propriétés d'un morceau de bois, dans lequel le morceau de bois est une partie sciée à partir d'une bille, ou un produit en bois formé par sciage, ou un produit en bois davantage traité, dans lequel un échantillon de bois est sélectionné à partir du morceau de bois, l'échantillon de bois est illuminé par une source de lumière, des signaux résultants sont observés et analysés à partir de l'échantillon de bois illuminé par la source de lumière, et une propriété de l'échantillon de bois est déterminée par les signaux, dans lequel l'échantillon de bois est une surface en coupe transversale d'une extrémité du morceau de bois; l'échantillon de bois est illuminé par une source de lumière ultraviolette et une source de lumière visible ; la lumière est focalisée sur la surface en coupe transversale du morceau de bois ; les signaux résultants sont observés et analysés à partir de l'échantillon de bois illuminé par la lumière ultraviolette sur la base de différences de fluorescence et à partir de l'échantillon de bois illuminé par la lumière visible sur la base de différences de couleur; et les propriétés sélectionnées dans le groupe de la pourriture, du champignon de pourriture, du bleuissement, du site de bois de cœur, de la quantité de bois de cœur, de l'emplacement de la moelle, de la quantité d'aubier, de l'emplacement du morceau de bois sur une bille et de leurs combinaisons sont déterminées à partir de l'échantillon de bois par les signaux de sorte que la pourriture, un champignon de pourriture et le bleuissement soient déterminés au moyen de signaux sur la base des différences de fluorescence et des différences de couleur, et que le site de bois de cœur, la quantité de bois de cœur, l'emplacement de la moelle, la quantité d'aubier et l'emplacement du morceau de bois sur une bille soient déterminés au moyen de signaux sur la base des différences de fluorescence.

2. Procédé selon la revendication 1, **caractérisé en ce que** l'échantillon de bois est illuminé séquentiellement par la source de lumière ultraviolette et la source de lumière visible.

3. Procédé selon la revendication 1 ou 2, **caractérisé en ce que** l'échantillon de bois est illuminé par de la lumière ultraviolette, et une fluorescence et des différences de fluorescence sont observées par un détecteur.

4. Procédé selon l'une quelconque des revendications 1 à 3, **caractérisé en ce que** l'échantillon de bois est illuminé par de la lumière ultraviolette, et une fluorescence et des différences de fluorescence sont observées par une caméra.

5. Procédé selon l'une quelconque des revendications 1 à 4, **caractérisé en ce que** l'échantillon de bois est illuminé par de la lumière visible, et des différences de couleur sont observées par une caméra.

6. Procédé selon l'une quelconque des revendications 1 à 5, **caractérisé en ce que** le signal est amplifié par un filtre.

7. Procédé selon l'une quelconque des revendications 1 à 6, **caractérisé en ce que** la longueur d'onde de la lumière ultraviolette est sélectionnée dans la plage de 300 à 450 nm.

8. Procédé selon l'une quelconque des revendications 1 à 7, **caractérisé en ce que** le procédé est utilisé dans le tri de morceaux de bois.

9. Appareil pour déterminer les propriétés d'un morceau de bois, dans lequel l'appareil comporte au moins une unité d'éclairage (1, 2) afin d'illuminer un échantillon de bois (7, 8) qui est le morceau de bois et dans lequel le morceau de bois est une partie sciée à partir d'une bille, ou un produit en bois formé par sciage, ou un produit en bois davantage traité, au moins des moyens d'observation (5, 6) afin d'observer et de mesurer les signaux résultants à partir de l'échantillon de bois illuminé (7, 8), des moyens d'analyse (9) afin d'analyser les signaux résultants, et des moyens de détermination (9) afin de déterminer les propriétés du morceau de bois sur la base des signaux, dans lequel
l'appareil comporte au moins une unité d'éclairage (1) comprenant une source de lumière basée sur de la lumière ultraviolette (3) et au moins une unité d'éclairage (2) comprenant une source de lumière basée sur de la lumière visible (4) afin d'illuminer l'échantillon de bois (7, 8) et afin de focaliser la lumière sur l'échantillon de bois qui est une surface en coupe transversale d'une extrémité du morceau de bois, des moyens d'analyse (9) sont agencés pour analyser les signaux résultants sur la base de différences de fluorescence dans l'échantillon de bois illuminé par la lumière ultraviolette et de différences de couleur dans l'échantillon de bois illuminé par la lumière visible, et des moyens de détermination (9) sont agencés pour déterminer les propriétés du morceau de bois sélectionnées dans le groupe de la pourriture, du champignon de pourriture, du bleuissement, du site de bois de cœur, de la quantité de bois de cœur, de l'emplacement de la moelle, de la quantité d'aubier, de l'emplacement du morceau de bois sur une bille et de leurs combinaisons sur la base des signaux de sorte que la pourriture, le champignon de pourriture et le bleuissement soient déterminés au moyen de signaux sur la base des différences de fluorescence et des différences de couleur, et que le site de bois de cœur, la quantité de bois de cœur, l'emplacement de la moelle, la quantité d'aubier, et l'emplacement du morceau de bois sur une bille soient déterminés au moyen de signaux sur la base de différences de fluorescence.

10. Appareil selon la revendication 9, **caractérisé en ce que** l'unité d'éclairage (1, 2) comporte 1 à 5 sources de lumière (3, 4).

11. Appareil selon la revendication 9 ou 10, **caractérisé en ce que** le moyen d'observation comporte 1 à 5 dispositifs d'observation (5, 6).

12. Appareil selon l'une quelconque des revendications 9 à 11, **caractérisé en ce que** l'appareil comporte un détecteur (5, 6) en tant que dispositif d'observation.

13. Appareil selon l'une quelconque des revendications 9 à 12, **caractérisé en ce que** l'appareil comporte une caméra (5, 6) en tant que dispositif d'observation.

14. Appareil selon l'une quelconque des revendications 9 à 13, **caractérisé en ce que** l'appareil comporte un filtre afin d'amplifier le signal.

15. Appareil selon l'une quelconque des revendications 9 à 14, **caractérisé en ce que** l'appareil comporte un logiciel informatique (9) en tant que moyens d'analyse et de détermination.
